# EUROPÄISCHE PATENTSCHRIFT

(11) **EP 3 772 342 B1**
(45) Veröffentlichungstag und Bekanntmachung des Hinweises auf die Patenterteilung: **28.07.2021**
(21) Anmeldenummer: 20187708.1
(22) Anmeldetag: 24.07.2020
(51) Int. Cl.: A61B 17/29, A61B 1/008, A61B 17/00

(54) **MEDIZINISCHES INSTRUMENT**
MEDICAL INSTRUMENT
INSTRUMENT MÉDICAL

(30) Priorität: 05.08.2019 DE 102019121088
(43) Veröffentlichungstag der Anmeldung: 10.02.2021
(73) Patentinhaber: Karl Storz SE & Co. KG, 78532 Tuttlingen (DE)
(72) Erfinder: Fauser, Heiner, 78532 Tuttlingen (DE); Kohli, Arno, 78532 Tuttlingen (DE); Grüner, Sven, 78532 Tuttlingen (DE)
(74) Vertreter: Hofmeister, Frank

(56) Entgegenhaltungen:
- WO-A2-2004/052171
- US-A1- 2013 218 141
- US-A1- 2015 164 524

## Beschreibung

Die Erfindung betrifft ein medizinisches Instrument mit einem hohlen Schaft, einer am proximalen Ende des Schaftes angeordneten Handhabe und einer am distalen Ende des Schaftes angeordneten Werkzeugspitze mit einem Werkzeug, wobei das Werkzeug über ein axial verschiebbar im Schaft gelagertes Betätigungselement betätigbar ist, das proximalseitig mit der Handhabe in Wirkverbindung steht und die Werkzeugspitze über einen Gelenkmechanismus relativ zur Längsachse des Schaftes verschwenkbar ist, wobei der Gelenkmechanismus aus am distalen Ende des Schaftes angeordneten Schwenkgliedern besteht, die über in Längsrichtung des Schaftes verlaufende Lenkdrähte so mit einem proximalseitigen Antrieb verbunden sind, dass eine Bewegung des proximalseitigen Antriebs eine entsprechende relative Bewegung der distalseitigen Schwenkglieder und somit ein Verschwenken der Werkzeugspitze verursacht, wobei im Inneren hohlen Schaftes zwischen den distalseitigen Schwenkgliedern und dem proximalseitigen Antrieb mindestens eine Führungsvorrichtung für die die Schwenkglieder mit dem Antrieb verbindenden Lenkdrähte angeordnet ist, wobei in der mindestens einen Führungsvorrichtung Aufnahmen für zumindest einige der Lenkdrähte ausgebildet sind.

Schwenkglieder mit drei, vier oder mehr außenliegenden Lenkdrähten/Lenkseilen für abwinkelbare medizinische Instrumente sind aus der Praxis für handgeführte und/oder robotische Instrumente bekannt. Für eine feinfühlige Steuerung des distalen Endes eines derartigen medizinischen Instruments haben sich viele dünne Lenkdrähte/Lenkseile gegenüber wenigen dickeren Lenkdrähten/Lenkseilen als vorteilhafter erwiesen, da so unter anderem eine gleichmäßigere Kraftverteilung in alle Abwinklungsrichtungen zu erzielen ist und darüber hinaus erlauben dünnere Lenkdrähte/Lenkseile mehr Platz im Innenraum für elektrische Leitungen und dergleichen.

Ein medizinisches Instrument mit über Lenkdrähten/Lenkseilen angesteuerten Schwenkgliedern ist beispielsweise aus der US 2013/0218141 A1 bekannt.

Die Verwendung vieler dünner Lenkdrähte/Lenkseile hat neben den genannten Vorteilen aber auch den Nachteil, dass die Montage deutlich erschwert wird, weil es sehr schwierig ist, alle Lenkdrähte/Lenkseile zusammen kreuzungsfrei und sortiert von distal durch das hohle Schaftrohr zu fädeln.

Aus der WO 2004/052171 A2 ist ein gattungsgemäßes medizinisches Instrument mit im Inneren des Schaftes angeordneten Führungsvorrichtungen für die Lenkdrähte bekannt. Diese bekannte Konstruktion ist in der Handhabung und Fertigung sehr kompliziert.

Davon ausgehend liegt der Erfindung die **Aufgabe** zugrunde, ein medizinisches Instrument der eingangs genannten Art so auszugestalten, dass eine einfache und kreuzungsfreie Montage der Lenkdrähte/Lenkseile gewährleistet ist.

Die **Lösung** dieser Aufgabenstellung ist erfindungsgemäß dadurch gekennzeichnet, dass die mindestens eine Führungsvorrichtung koaxial auf dem Betätigungselement angeordnet ist und, dass die mindestens eine Führungsvorrichtung verdrehsicher auf dem Betätigungselement gelagert ist.

Durch die Verwendung einer zwischen den distalseitigen Schwenkgliedern und dem proximalseitigen Antrieb im Inneren des hohlen Schaftrohres angeordneten Führungsvorrichtung für die Lenkdrähte ist es möglich, den Bereich zwischen den distalseitigen Schwenkgliedern und dem proximalseitigen Antrieb zu überbrücken, so dass ein kreuzungsfreies Einführen der Lenkdrähte von distal nach proximal gewährleistet ist. Die Anordnung der Führungsvorrichtung im Inneren des Schaftes kann dabei alternativ an der Innenseite des hohlen Schaftrohres oder als in das Schaftrohr einsetzbare Vorrichtung ausgebildet sein.

Die Anordnung der mindestens einen Führungsvorrichtung für die Lenkdrähte koaxial auf dem zentral im Schaftrohr verlaufenden Betätigungselement ermöglicht eine einfache Vorkonfektionierung des bei der Montage von distal in das hohle Schaftrohr einzuführenden Betätigungselements mit den kreuzungsfrei geordnet über die mindestens eine Führungsvorrichtung verlaufenden Lenkdrähten.

Die Verdrehsicherheit der Führungsvorrichtung soll gewährleisten, dass die in der mindestens einen Führungsvorrichtung angeordneten Lenkdrähte sich nicht wendelförmig um das zentrale Betätigungselement verdrehen können.

Zur Ausbildung der Verdrehsicherheit sind gemäß der Erfindung an der Führungsvorrichtung und am Betätigungselement eine oder mehrere miteinander korrespondierende unrunde Anlageflächen ausgebildet.

Weiterhin wird erfindungsgemäß vorgeschlagen, dass die mindestens eine Führungsvorrichtung zusätzlich zu den Aufnahmen für die Lenkdrähte mindestens eine Spülöffnung aufweist. Die mindestens eine Spülöffnung in der Führungsvorrichtung soll sicherstellen, dass beim Spülen des Schaftrohres auch die im Inneren des Schaftrohres angeordneten Führungsvorrichtungen gut umströmt und somit gut gereinigt werden können.

Gemäß einer praktischen Ausführungsform zur Ausbildung der Führungsvorrichtung wird erfindungsgemäß vorgeschlagen, dass die mindestens eine Führungsvorrichtung als quer zur Längsachse des Schaftes angeordneter scheibenförmiger Ring ausgebildet ist. Die Ausbildung als Ringscheibe stellt eine einfach herzustellende und stabile Ausgestaltungsform der Führungsvorrichtung dar.

Um insbesondere bei langen Schäften die geordnete und kreuzungsfreie Verlegung der Lenkdrähte zu gewährleisten, wird mit einer praktischen Ausführungsform der Erfindung vorgeschlagen, dass mindestens zwei Führungsvorrichtungen mit Abstand zueinander auf dem Betätigungselement angeordnet sind. Durch die Verwendung von zwei oder mehr Führungsvorrichtungen wird die Montage weiter vereinfacht, da die Gefahr einer Kreuzung der Lenkdrähte mit jeder zusätzlichen Führungsvorrichtung verringert wird.

Zur Ausbildung der Aufnahmen für die Lenkdrähte wird gemäß einer ersten Ausführungsform der Erfindung vorgeschlagen, dass die in der mindestens einen Führungsvorrichtung ausgebildeten Aufnahmen für die Lenkdrähte als Durchgangsbohrungen ausgebildet sind. Die Ausbildung der Aufnahmen als Durchgangsbohrungen hat den Vorteil, dass die einmal eingefädelten Lenkdrähte dauerhaft sicher geführt werden.

Gemäß einer alternativen Ausführungsform zur Ausbildung der Aufnahmen für die Lenkdrähte wird mit der Erfindung vorgeschlagen, dass die in der mindestens einen Führungsvorrichtung ausgebildeten Aufnahmen für die Lenkdrähte als nach außen offene radial nach innen verlaufende Schlitze ausgebildet sind. Die Ausbildung der Aufnahmen als nach außen offene Schlitze ermöglicht ein schnelles und einfaches Einlegen eines jeden Lenkdrahts in die zugehörige Aufnahme der Führungsvorrichtung.

Mit einer ersten praktischen Ausführungsform zur Ausbildung der scheibenförmigen Führungsvorrichtung wird vorgeschlagen, dass die als scheibenförmiger Ring ausgebildete Führungsvorrichtung mindestens einen mit den Aufnahmen für die Lenkdrähte versehenen Teilbereich und mindestens einen mit einer Spülöffnung versehenen Teilbereich aufweist.

Gemäß einer alternativen zweiten Ausführungsform wird erfindungsgemäß vorgeschlagen, dass die als scheibenförmiger Ring ausgebildete Führungsvorrichtung zwei mit den Aufnahmen für die Lenkdrähte versehene Teilbereiche und zwei mit Spülöffnungen versehene Teilbereiche aufweist, wobei die jeweiligen Teilbereiche versetzt zueinander angeordnet sind.

Schließlich wird mit der Erfindung vorgeschlagen, dass bei der Verwendung von mindestens zwei auf dem Betätigungselement angeordneten, als scheibenförmiger Ring ausgebildeten Führungsvorrichtungen, jeweils zwei in Richtung der Längsachse des Schaftes direkt hintereinander angeordnete Führungsvorrichtungen so zueinander angeordnet sind, dass mit den Aufnahmen für die Lenkdrähte versehene Teilbereiche und mit Spülöffnungen versehene Teilbereiche versetzt zueinander angeordnet sind. Durch die versetzte Anordnung der mit den Aufnahmen versehenen Teilbereiche und der mit den Spülöffnungen versehenen Teilbereiche zueinander wird sichergestellt, dass einerseits alle Lenkdrähte durch Teilbereiche der hintereinander angeordneten Führungsvorrichtungen kreuzungsfrei geführt werden und andererseits eine durch die Spülöffnungen fließende Spülflüssigkeit mehrfach umgelenkt wird und so eine vollständige Durchspülung des hohlen Schaftrohres gewährleistet ist.

Weitere Merkmale und Vorteile der Erfindung ergeben sich anhand der zugehörigen Zeichnungen, in denen verschiedene Ausführungsbeispiele eines erfindungsgemäßen medizinischen Instruments nur beispielhaft dargestellt sind, ohne die Erfindung auf diese Ausführungsbeispiele zu beschränken. In den Zeichnungen zeigt:
- Fig. 1: eine perspektivische Seitenansicht eines medizinischen Instruments gemäß dem Stand der Technik;
- Fig. 2: eine vergrößerte perspektivische Ansicht des Bereichs II gemäß Fig. 1, jedoch ein erfindungsgemäßes medizinisches Instrument ohne Schaftrohr darstellend;
- Fig. 3: eine perspektivische Ansicht des erfindungsgemäßen medizinischen Instruments ohne Schaftrohr gemäß Fig. 2 von proximal, jedoch eine alternative Ausführungsform der Führungsvorrichtungen darstellend;
- Fig. 4: eine Ansicht gemäß Fig. 3, jedoch eine weitere alternative Ausführungsform der Führungsvorrichtungen darstellend;
- Fig. 5: einen vergrößerten Schnitt entlang der Linie V-V gemäß Fig. 4 und
- Fig. 6: eine Schnittdarstellung gemäß Fig. 5, jedoch eine weitere alternative Ausführungsform der Führungsvorrichtungen darstellend.

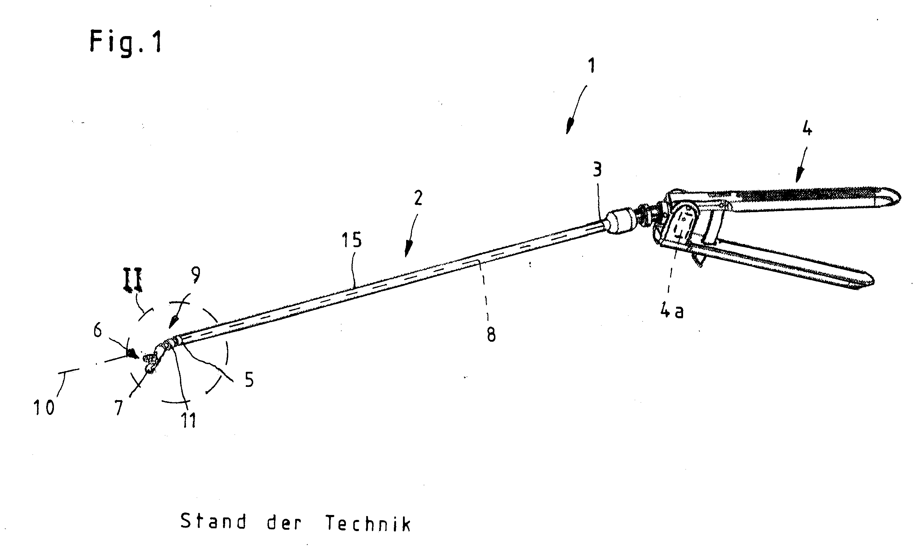

Die Abbildung Fig. 1 zeigt ein medizinisches Instrument 1 mit einem hohlen Schaft 2, einer am proximalen Ende 3 des Schaftes 2 angeordneten Handhabe 4 und einer am distalen Ende 5 des Schaftes 2 angeordneten Werkzeugspitze 6 mit einem Werkzeug 7, wobei das Werkzeug 7 über ein axial verschiebbar im Schaft 2 gelagertes Betätigungselement 8 betätigbar ist, das proximalseitig mit der Handhabe 4 in Wirkverbindung steht.

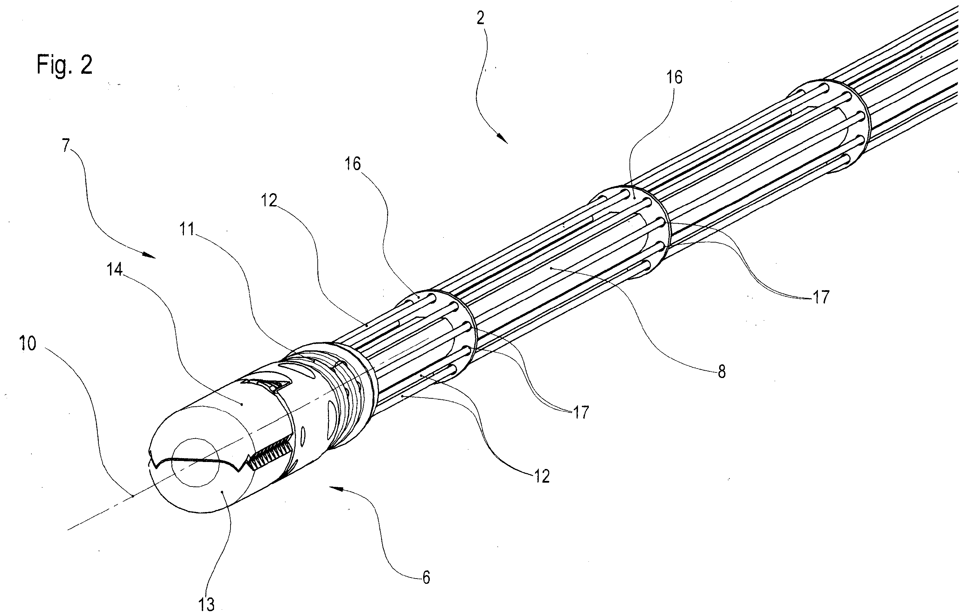

Die Werkzeugspitze 6 ist über einen Gelenkmechanismus 9 relativ zur Längsachse 10 des Schaftes 2 verschwenkbar, wobei der Gelenkmechanismus 9 aus am distalen Ende des Schaftes 5 angeordneten Schwenkgliedern 11 besteht, die über in Längsrichtung des Schaftes 2 verlaufende Lenkdrähte 12 (Fig. 2-4) so mit einem proximalseitigen Antrieb 4a verbunden sind, dass eine Bewegung des proximalseitigen Antriebs 4a eine entsprechende relative Bewegung der distalseitigen Schwenkglieder 11 und somit ein Verschwenken der Werkzeugspitze 6 verursacht.

Auch wenn voranstehend und nachfolgend nur der Begriff Lenkdrähte 12 verwendet wird, können funktional auch Lenkseile verwendet werden, weshalb der verwendete Begriff Lenkdrähte 12 synonym auch als Lenkseil zu lesen und zu verstehen ist.

Das axialverschiebbar im Schaft 2 gelagerte Betätigungselement 8 zum Betätigen des beispielsweise aus zwei Maulteilen 13 und 14 (Fig. 2) bestehenden Werkzeugs 7, ist bei den dargestellten Ausführungsformen als Zug-/Schubstange ausgebildet.

Bei der Montage des medizinischen Instruments 1 wird in einem ersten Arbeitsschritt das distale Ende des medizinischen Instruments mit der Werkzeugspitze 6, den distalseitigen Schwenkgliedern 11 und dem distalseitig mit der Werkzeugspitze 6 verbundenen Betätigungselement 8 sowie den an den distalseitigen Schwenkgliedern 11 gelagerten Lenkdrähten 12 zusammengefügt. Im nachfolgenden Montageschritt müssen die Lenkdrähte 12 und das Betätigungselement 8 von distal in ein hohles Schaftrohr 15 des Schaftes 2 eingeführt werden.

Insbesondere bei der Verwendung vieler dünner Lenkdrähte 12 ist es sehr schwierig, alle Lenkdrähte 12 zusammen kreuzungsfrei und sortiert von distal durch das hohle Schaftrohr 15 zu fädeln.

Um einen kreuzungsfreien Verlauf der Lenkdrähte 12 zwischen den distalseitigen Schwenkgliedern 11 und dem proximalseitigen Antrieb 4a durch das hohle Schaftrohr 15 hindurch zu gewährleisten, ist bei den in den Abbildungen Fig. 2 bis 4 dargestellten Ausführungsformen im Inneren hohlen Schaftes 2 zwischen den distalseitigen Schwenkgliedern 11 und dem proximalseitigen Antrieb 4a mindestens eine Führungsvorrichtung 16 für die die Schwenkglieder 11 mit dem Antrieb 4a verbindenden Lenkdrähte 12 angeordnet, wobei in der mindestens einen Führungsvorrichtung 16 Aufnahmen 17 für zumindest einige der Lenkdrähte 12 ausgebildet sind.

Wie aus den Abbildungen Fig. 2 bis 4 weiterhin ersichtlich, sind über die Länge des Schaftes 2 vorteilhafterweise mehrere Führungsvorrichtungen 16 mit Abstand zueinander hintereinander entlang der Längsachse 10 des Schaftes 2 angeordnet, um das Einfädeln in das hohle Schaftrohr 15 zu erleichtern.

Bei den dargestellten Ausführungsformen sind die Führungsvorrichtungen 16 als koaxial auf dem Betätigungselement 8 und quer zur Längsachse 10 des Schaftes 2 angeordnete scheibenförmige Ringe ausgebildet.

Alternativ ist es auch möglich, die Führungsvorrichtung 16 auf der Innenseite der beiderseitigen Enden des hohlen Schaftrohres 15 anzuordnen.

Die Anordnung der Führungsvorrichtungen 16 auf dem Betätigungselement 8 hat den Vorteil, dass das Einführen der Lenkdrähte 12 in die Aufnahmen 17 sehr einfach und schnell erfolgen kann und darüber hinaus das Betätigungselement 8 mit den Führungsvorrichtungen 16 und den in den Aufnahmen 17 der Führungsvorrichtungen 16 angeordneten Lenkdrähten 12 vollständig vorkonfektioniert werden kann, bevor dieses dann von distal in das hohle Schaftrohr 15 eingeführt wird.

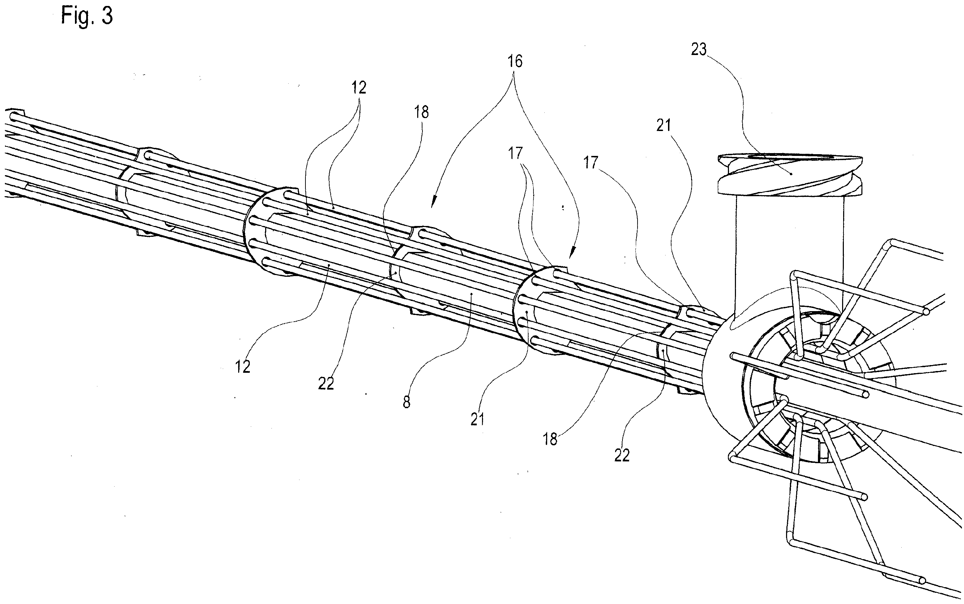

Bei den in den Abbildungen Fig. 3 bis 6 dargestellten Ausführungsformen zur Ausbildung der Führungsvorrichtungen 16 weisen die Führungsvorrichtungen 16 zusätzlich zu den Aufnahmen 17 für die Lenkdrähte 12 mindestens eine Spülöffnung 18 auf. Die mindestens eine Spülöffnung 18 in der Führungsvorrichtung 16 soll beim Spülen des Schaftrohres 15 ein Umströmen der Führungsvorrichtungen 16 und somit ein vollständiges Reinigen des Schaftrohres 15 erleichtern.

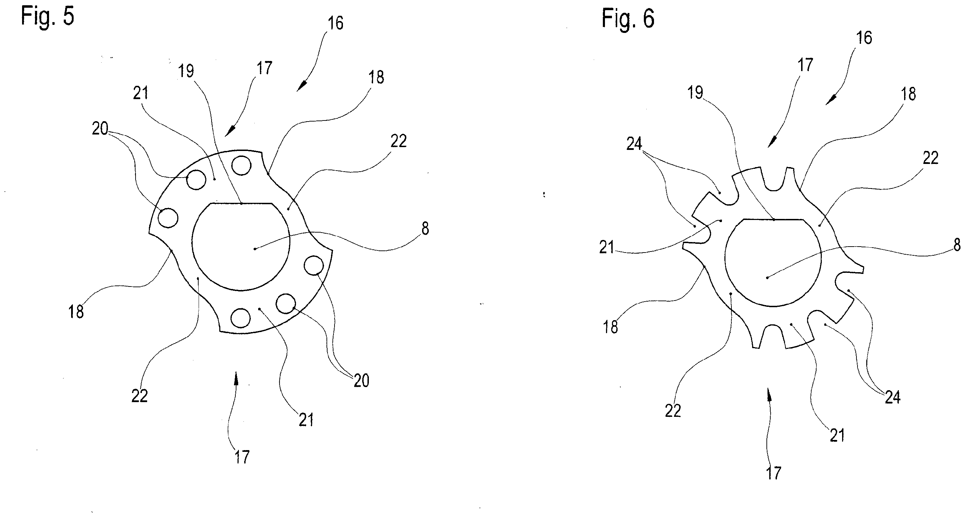

Damit sich die in den Führungsvorrichtungen 16 angeordneten Lenkdrähte 12 nicht wendelförmig um das zentrale Betätigungselement 8 verdrehen können, sind die Führungsvorrichtungen 16 vorteilhafterweise verdrehsicher auf dem Betätigungselement 8 gelagert. Zur Ausbildung der Verdrehsicherheit sind, wie aus den Abbildungen Fig. 5 und 6 ersichtlich, an der Führungsvorrichtung 16 und am Betätigungselement 8 miteinander korrespondierende unrunde Anlageflächen 19 ausgebildet.

Alternativ zur Ausbildung der unrunden Anlageflächen 19 ist es selbstverständlich auch möglich, die Führungsvorrichtungen 16 beispielsweise durch Verschweißen oder Verpressen verdrehsicher auf dem Betätigungselement 8 festzulegen.

Bei der in Fig. 2 dargestellten Ausführungsform der Führungsvorrichtung 16 ist die Führungsvorrichtung 16 als vollständiger scheibenförmiger Ring ausgebildet, in dem für jeden Lenkdraht 12 eine als Durchgangsbohrung 20 ausgebildete Aufnahme 17 ausgebildet ist.

Die in den Abbildungen Fig. 3 bis 6 dargestellten Ausführungsformen zur Ausbildung der Führungsvorrichtungen 16 zeichnen sich dadurch aus, dass die scheibenförmigen Ringe Teilbereiche 21 mit Aufnahmen 17 für die Lenkdrähte 12 und Teilbereiche 22 für die Spülöffnung 19 aufweisen.

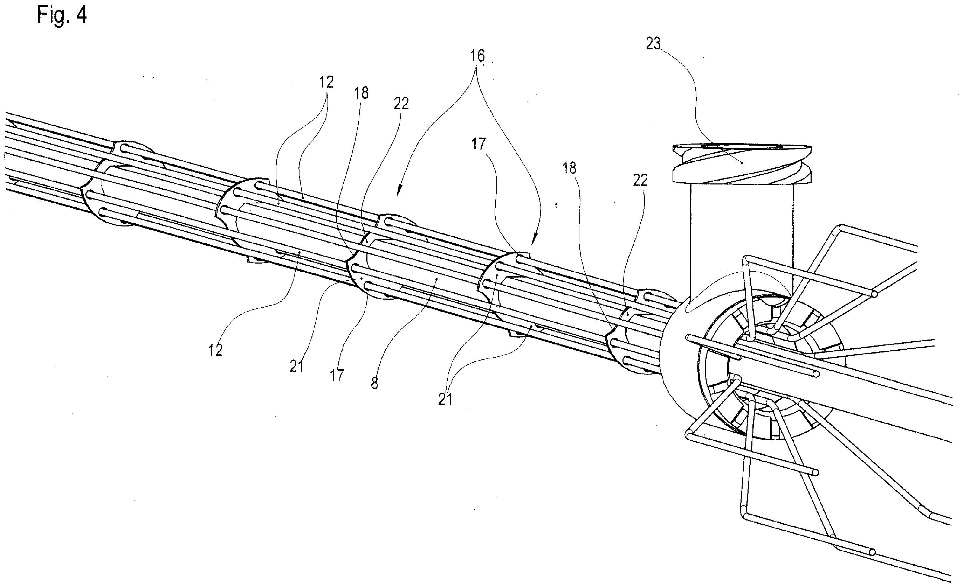

Wenn mehrere Führungsvorrichtungen 16 hintereinander auf dem Betätigungselement 8 angeordnet sind, wie dies in den Abbildungen Fig. 3 und 4 dargestellt ist, ist es für eine sicher und kreuzungsfreie Führung der Lenkdrähte 12 ausreichend, wenn der einzelne Lenkdraht 12 nur in jeder zweiten oder dritten Führungsvorrichtung 16 geführt wird.

Während bei der in Fig. 3 dargestellten Ausführungsform jede Führungsvorrichtung 16 jeweils einen Teilbereich 21 mit Aufnahmen 17 für die Lenkdrähte 12 und jeweils einen Teilbereich 22 für die Spülöffnung 18 aufweist, sind es bei den in den Abbildungen Fig. 4 bis 6 dargestellten Ausführungsformen jeweils zwei Teilbereiche 21 mit Aufnahmen 17 für die Lenkdrähte 12 und jeweils zwei Teilbereiche 22 für die Spülöffnung 18.

Wie weiterhin aus den Abbildungen Fig. 3 und 4 ersichtlich, sind jeweils zwei in Richtung der Längsachse 10 des Schaftes 2 direkt hintereinander angeordnete Führungsvorrichtungen 16 so zueinander angeordnet, dass mit den Aufnahmen 17 für die Lenkdrähte 12 versehene Teilbereiche 21 und mit Spülöffnungen 18 versehene Teilbereiche 22 versetzt zueinander angeordnet sind.

Diese versetzte Anordnung der Teilbereiche 21 und 22 der Führungsvorrichtungen 16 zueinander bewirkt, dass eine über einen proximalen Spülanschluss 23 in das hohle Schaftrohr 15 eingespeist Spülflüssigkeit mäanderförmig die Führungsvorrichtungen 16 umströmen muss. Die so entstehende turbulente Strömung stellt eine Verbesserung der Reinigungswirkung gegenüber dem geraden Durchspülen dar.

Alternativ zu den dargestellten Ausführungsformen sind selbstverständlich auch Führungsvorrichtungen mit mehr als zwei Teilbereichen 21 und 22 verwendbar.

Die Abbildung Fig. 6 zeigt schließlich eine alternative Ausführungsform zur Ausbildung der Aufnahmen 17 zum Führen der Lenkdrähte 12. Bei dieser Ausführungsform sind die Aufnahmen 17 als nach außen offene radial nach innen verlaufende Schlitze 24 ausgebildet. Diese Ausbildung ermöglicht ein schnelles und einfaches Einlegen eines jeden Lenkdrahts 12 in die zugehörige Aufnahme 17 der Führungsvorrichtung 16.

Ein wie zuvor ausgebildetes medizinisches Instrument 1 zeichnet sich dadurch aus, dass durch die Verwendung der Führungsvorrichtungen 16 jeder Lenkdraht 12 von distal nach proximal sicher geführt wird, so dass zu keinem Zeitpunkt die Gefahr besteht, vertauscht oder überkreuzt zu werden.

Ein positiver Nebeneffekt dieses geführten Aufbaus ist, dass die Lenkdrähte 12 auch im Betrieb nicht aneinander reiben und sich im Lenkverhalten nicht gegenseitig beeinflussen. Weiterhin hängen die zwischen den distalen Schwenkgliedern 11 und dem proximalen Antrieb 4a mittels der Führungsvorrichtungen 16 geführten Lenkdrähte 12 nicht durch und können so direkter auf Zug reagieren.

Die Verwendung der im Inneren des Schaftes 2 angeordneten Führungsvorrichtungen 16 für die Lenkdrähte 12 gewährleistet eine einfache und kreuzungsfreie Montage der Lenkdrähte 12.

### Bezugszeichenliste

- 1: medizinisches Instrument
- 2: Schaft
- 3: proximales Ende (Schaft)
- 4: Handhabe
- 4a: Antrieb
- 5: distales Ende (Schaft)
- 6: Werkzeugspitze
- 7: Werkzeug
- 8: Betätigungselement
- 9: Gelenkmechanismus
- 10: Längsachse
- 11: Schwenkglied
- 12: Lenkdraht
- 13: Maulteil
- 14: Maulteil
- 15: Schaftrohr
- 16: Führungsvorrichtung
- 17: Aufnahme
- 18: Spülöffnung
- 19: Anlagefläche
- 20: Durchgangsbohrung
- 21: Teilbereich
- 22: Teilbereich
- 23: Spülanschluss
- 24: Schlitz

## Patentansprüche

1. Medizinisches Instrument mit einem hohlen Schaft (2), einer am proximalen Ende (3) des Schaftes (2) angeordneten Handhabe (4) und einer am distalen Ende (5) des Schaftes (2) angeordneten Werkzeugspitze (6) mit einem Werkzeug (7), wobei das Werkzeug (7) über ein axial verschiebbar im Schaft (2) gelagertes Betätigungselement (8) betätigbar ist, das proximalseitig mit der Handhabe (4) in Wirkverbindung steht und die Werkzeugspitze (6) über einen Gelenkmechanismus (9) relativ zur Längsachse (10) des Schaftes (2) verschwenkbar ist, wobei der Gelenkmechanismus (9) aus am distalen Ende (5) des Schaftes (2) angeordneten Schwenkgliedern (11) besteht, die über in Längsrichtung des Schaftes (2) verlaufende Lenkdrähte (12) so mit einem proximalseitigen Antrieb (4a) verbunden sind, dass eine Bewegung des proximalseitigen Antriebs (4a) eine entsprechende relative Bewegung der distalseitigen Schwenkglieder (11) und somit ein Verschwenken der Werkzeugspitze (6) verursacht, wobei im Inneren hohlen Schaftes (2) zwischen den distalseitigen Schwenkgliedern (11) und dem proximalseitigen Antrieb (4a) mindestens eine Führungsvorrichtung (16) für die die Schwenkglieder (11) mit dem Antrieb (4a) verbindenden Lenkdrähte (12) angeordnet ist, wobei in der mindestens einen Führungsvorrichtung (16) Aufnahmen (17) für zumindest einige der Lenkdrähte (12) ausgebildet sind,
**dadurch gekennzeichnet,**
**dass** die mindestens eine Führungsvorrichtung (16) koaxial auf dem Betätigungselement (8) angeordnet ist und, dass die mindestens eine Führungsvorrichtung (16) verdrehsicher auf dem Betätigungselement (8) gelagert ist.

2. Medizinisches Instrument nach Anspruch 1, **dadurch gekennzeichnet, dass** an der Führungsvorrichtung (16) und am Betätigungselement (8) eine oder mehrere miteinander korrespondierende unrunde Anlageflächen (19) als Verdrehsicherung ausgebildet sind.

3. Medizinisches Instrument nach Anspruch 1 oder 2, **dadurch gekennzeichnet, dass** das Betätigungselement (8) als Zug-/Schubstange ausgebildet ist.

4. Medizinisches Instrument nach einem der Ansprüche 1 bis 3, **dadurch gekennzeichnet, dass** die mindestens eine Führungsvorrichtung (16) zusätzlich zu den Aufnahmen (17) für die Lenkdrähte (12) mindestens eine Spülöffnung (18) aufweist.

5. Medizinisches Instrument nach einem der Ansprüche 1 bis 4, **dadurch gekennzeichnet, dass** die mindestens eine Führungsvorrichtung (16) als quer zur Längsachse (10) des Schaftes (2) angeordneter scheibenförmiger Ring ausgebildet ist.

6. Medizinisches Instrument nach einem der Ansprüche 2 bis 5, **dadurch gekennzeichnet, dass** mindestens zwei Führungsvorrichtungen (16) mit Abstand zueinander auf dem Betätigungselement (8) angeordnet sind.

7. Medizinisches Instrument nach einem der Ansprüche 1 bis 6, **dadurch gekennzeichnet, dass** die in der mindestens einen Führungsvorrichtung (16) ausgebildeten Aufnahmen (17) für die Lenkdrähte (12) als Durchgangsbohrungen (20) ausgebildet sind.

8. Medizinisches Instrument nach einem der Ansprüche 1 bis 6, **dadurch gekennzeichnet, dass** die in der mindestens einen Führungsvorrichtung (16) ausgebildeten Aufnahmen (17) für die Lenkdrähte (12) als nach außen offene radial nach innen verlaufende Schlitze (24) ausgebildet sind.

9. Medizinisches Instrument nach einem der Ansprüche 5 bis 8, **dadurch gekennzeichnet, dass** die als scheibenförmiger Ring ausgebildete Führungsvorrichtung (16) mindestens einen mit den Aufnahmen (17) für die Lenkdrähte (12) versehenen Teilbereich (21) und mindestens einen mit einer Spülöffnung (18) versehenen Teilbereich (22) aufweist.

10. Medizinisches Instrument nach Anspruch 9, **dadurch gekennzeichnet, dass** die als scheibenförmiger Ring ausgebildete Führungsvorrichtung (16) zwei mit den Aufnahmen (17) für die Lenkdrähte (12) versehene Teilbereiche (21) und zwei mit Spülöffnungen (18) versehene Teilbereiche (22) aufweist, wobei die jeweiligen Teilbereiche (21, 22) versetzt zueinander angeordnet sind.

11. Medizinisches Instrument nach Anspruch 9 oder 10 mit mindestens zwei auf dem Betätigungselement (8) angeordneten, als scheibenförmiger Ring ausgebildeten Führungsvorrichtungen (16), **dadurch gekennzeichnet, dass** jeweils zwei in Richtung der Längsachse (10) des Schaftes (2) direkt hintereinander angeordnete Führungsvorrichtungen (16) so zueinander angeordnet sind, dass mit den Aufnahmen (17) für die Lenkdrähte (12) versehene Teilbereiche (21) und mit Spülöffnungen (18) versehene Teilbereiche (22) versetzt zueinander angeordnet sind.

## Claims

1. Medical instrument with a hollow shaft (2), a handle (4) arranged at the proximal end (3) of the shaft (2), and a tool tip (6) with a tool (7) arranged at the distal end (5) of the shaft (2), wherein the tool (7) can be actuated via an actuation element (8) which is mounted axially displaceably in the shaft (2) and which is operatively connected at the proximal end to the handle (4), and the tool tip (6) is pivotable relative to the longitudinal axis (10) of the shaft (2) via a joint mechanism (9), wherein the joint mechanism (9) is composed of pivoting members (11) which are arranged at the distal end (5) of the shaft (2) and which are connected to a proximal-side drive (4a), via steering wires (12) running in the longitudinal direction of the shaft (2), in such a way that a movement of the proximal-side drive (4a) causes a corresponding relative movement of the distal-side pivoting members (11) and thus a pivoting of the tool tip (6), wherein in the interior of the hollow shaft (2), between the distal-side pivoting members (11) and the proximal-side drive (4a), at least one guide device (16) is arranged for the steering wires (12) connecting the pivoting members (11) to the drive (4a), wherein receptacles (17) for at least some of the steering wires (12) are formed in the at least one guide device (16), **characterized in that** the at least one guide device (16) is arranged coaxially on the actuation element (8), and **in that** the at least one guide device (16) is mounted on the actuation element (8) in a manner secure against rotation.

2. Medical instrument according to Claim 1, **characterized in that** one or more non-circular contact surfaces (19) corresponding to one another are formed on the guide device (16) and on the actuation element (8) in order to prevent rotation.

3. Medical instrument according to Claim 1 or 2, **characterized in that** the actuation element (8) is designed as a pull/push rod.

4. Medical instrument according to one of Claims 1 to 3, **characterized in that** the at least one guide device (16) has at least one rinsing opening (18) in addition to the receptacles (17) for the steering wires (12).

5. Medical instrument according to one of Claims 1 to 4, **characterized in that** the at least one guide device (16) is designed as a disc-shaped ring that is arranged transversely with respect to the longitudinal axis (10) of the shaft (2).

6. Medical instrument according to one of Claims 2 to 5, **characterized in that** at least two guide devices (16) are arranged spaced apart from one another on the actuation element (8).

7. Medical instrument according to one of Claims 1 to 6, **characterized in that** the receptacles (17) formed for the steering wires (12) in the at least one guide device (16) are designed as through-holes (20).

8. Medical instrument according to one of Claims 1 to 6, **characterized in that** the receptacles (17) formed in the at least one guide device (16) for the steering wires (12) are designed as radial inwardly extending slots (24) that are open to the outside.

9. Medical instrument according to one of Claims 5 to 8, **characterized in that** the guide device (16) designed as a disc-shaped ring comprises at least one subregion (21) provided with the receptacles (17) for the steering wires (12) and at least one subregion (22) provided with a rinsing opening (18) .

10. Medical instrument according to Claim 9, **characterized in that** the guide device (16) designed as a disc-shaped ring has two subregions (21) provided with the receptacles (17) for the steering wires (12) and two subregions (22) provided with rinsing openings (18), wherein the respective subregions (21, 22) are arranged offset from each other.

11. Medical instrument according to Claim 9 or 10, with at least two guide devices (16), designed as a disc-shaped ring, arranged on the actuation element (8), **characterized in that** in each case two guide devices (16) arranged directly one behind the other in the direction of the longitudinal axis (10) of the shaft (2) are arranged relative to each other such that subregions (21) provided with the receptacles (17) for the steering wires (12) and subregions (22) provided with rinsing openings (18) are arranged offset from each other.

## Revendications

1. Instrument médical comprenant une tige creuse (2), une poignée (4) disposée à l'extrémité proximale (3) de la tige (2) et une pointe d'outil (6) pourvue d'un outil (7) et disposée à l'extrémité distale (5) de la tige (2), l'outil (7) pouvant être actionné via un élément d'actionnement (8) qui est monté de manière à pouvoir coulisser axialement dans tige (2) et qui est relié fonctionnellement du côté proximal à la poignée (4) et la pointe d'outil (6) pouvant pivoter via un mécanisme à charnière (9) par rapport à l'axe longitudinal (10) de la tige (2), le mécanisme d'articulation (9) étant constitué des éléments de pivotement (11) qui sont disposés à l'extrémité distale (5) de la tige (2) et qui sont reliés à un entraînement côté proximal (4a) via des fils de direction (12) s'étendant dans la direction longitudinale de la tige (2), de sorte qu'un mouvement de l'entraînement côté proximal (4a) provoque un mouvement relatif correspondant des éléments de pivotement côté distal (11) et donc un pivotement de la pointe d'outil (6), au moins un dispositif de guidage (16) destiné aux fils de direction (12), qui relient les éléments de pivotement (11) à l'entraînement (4a), étant disposé à l'intérieur de la tige creuse (2) entre les éléments de pivotement côté distal (11) et l'entraînement côté proximal (4a), des logements (17) destiné au moins à certains des fils de direction (12) étant formés dans l'au moins un dispositif de guidage (16),
**caractérisé en ce que**
l'au moins un dispositif de guidage (16) est disposé coaxialement sur l'élément d'actionnement (8) et **en ce que** l'au moins un dispositif de guidage (16) est monté solidairement en rotation sur l'élément d'actionnement (8) .

2. Instrument médical selon la revendication 1, **caractérisé en ce qu'**une ou plusieurs surfaces d'appui non rondes (19) correspondant les unes aux autres sont formées au niveau du dispositif de guidage (16) et au niveau de l'élément d'actionnement (8) pour empêcher la rotation.

3. Instrument médical selon la revendication 1 ou 2, **caractérisé en ce que** l'élément d'actionnement (8) est réalisé sous la forme d'une tige de traction-poussée.

4. Instrument médical selon l'une des revendications 1 à 3, **caractérisé en ce que** l'au moins un dispositif de guidage (16) comporte au moins une ouverture de rinçage (18) en plus des logements (17) destinés aux fils de direction (12).

5. Instrument médical selon l'une des revendications 1 à 4, **caractérisé en ce que** l'au moins un dispositif de guidage (16) est réalisé sous la forme d'une bague en forme de disque disposée transversalement à l'axe longitudinal (10) de la tige (2).

6. Instrument médical selon l'une des revendications 2 à 5, **caractérisé en ce qu'**au moins deux dispositifs de guidage (16) sont disposés à distance l'un de l'autre sur l'élément d'actionnement (8).

7. Instrument médical selon l'une des revendications 1 à 6, **caractérisé en ce que** les logements (17) formés dans l'au moins un dispositif de guidage (16) destiné aux fils de direction (12) sont réalisés sous la forme d'alésages traversants (20).

8. Instrument médical selon l'une des revendications 1 à 6, **caractérisé en ce que** les logements (17) formés dans l'au moins un dispositif de guidage (16) destinés aux fils de direction (12) sont réalisés sous la forme de fentes (24) ouvertes vers l'extérieur et s'étendant radialement vers l'intérieur.

9. Instrument médical selon l'une des revendications 5 à 8, **caractérisé en ce que** le dispositif de guidage (16) réalisé sous la forme d'une bague en forme de disque comporte au moins une partie (21) pourvue des logements (17) destinés aux fils de direction (12) et au moins une partie (22) pourvue d'une ouverture de rinçage (18).

10. Instrument médical selon la revendication 9, **caractérisé en ce que** le dispositif de guidage (16) réalisé sous la forme d'une bague en forme de disque comporte deux parties (21) pourvues des logements (17) destinés aux fils de direction (12) et deux parties (22) pourvues d'ouvertures de rinçage (18), les parties respectives (21, 22) étant disposées de manière décalée les unes par rapport aux autres.

11. Instrument médical selon la revendication 9 ou 10 comprenant au moins deux dispositifs de guidage (16) disposés sur l'élément d'actionnement (8) et réalisés sous la forme d'une bague en forme de disque, **caractérisé en ce que** deux dispositifs de guidage (16), disposés directement l'un derrière l'autre dans la direction de l'axe longitudinal (10) de la tige (2), sont disposés l'un par rapport à l'autre de telle sorte que les parties (21) pourvues des logements (17) destinés aux fils de direction (12) et les parties (22) pourvues d'ouvertures de rinçage (18) soient disposées de manière décalée les unes aux autres.
